# EUROPEAN PATENT APPLICATION

(11) **EP 4 628 072 A1**
(43) Date of publication of application: **08.10.2025**
(21) Application number: 23898050.2
(22) Date of filing: 06.10.2023
(51) Int. Cl.: A61K 31/05, A61K 47/44, A61P 15/08

(54) **COMPOSITION FOR IMPROVING REPRODUCTIVE ABILITY OF LIVESTOCK**

(30) Priority: 02.12.2022 KR 20220166454; 15.03.2023 KR 20230033816
(71) Applicant: Solomon Co., Ltd., Sangju-si, Gyeongsangbuk-do 37224 (KR)
(72) Inventor: YOON, Minjung, Yongin-si, Gyeonggi-do 16853 (KR); SONG, Yu Bin, Yongin-si Gyeonggi-do 16979 (KR)
(74) Representative: Haseltine Lake Kempner LLP
(86) International application number: PCT/KR2023/015390
(87) International publication number: WO 2024/117516

(57) **Abstract**

The present invention relates to a composition for improving the reproductive ability of livestock, and can enhance productivity through improved reproductive ability of livestock and prevent various problems that may arise from indiscriminate use of hormonal preparations, and can alleviate economic burden of breeders, such as feed costs.

## Description

### Technical Field

The present invention relates to a composition for improving reproductive performance of livestock.

### Background Art

Livestock farms frequently experience reduced productivity due to reproductive disorders in female animals after parturition, which include delayed return to estrus, increased frequency of weak or silent estrus, irregular estrous cycles, *etc.* In particular, despite the decrease in reproduction rates, production costs such as feed expenses remain the same, leading to an increased financial burden on farmers.

In order to solve these problems, most farms inject synthetic GnRH preparations to induce estrus in livestock; however, the development of resistance due to continuous use necessitates increasing doses. This escalating use raises concerns among consumers regarding livestock intended for food, and it also poses a problem due to the potential intake of residual drugs through meat consumption. Additionally, the continuous and indiscriminate injection of hormonal agents can cause stress in livestock, increase the workload for farmers, and pose safety risks to workers during administration. Additionally, even when estrus is induced using injections, the optimal timing for insemination is often missed, and thus there is a limit to ideally increasing the actual success rate of artificial insemination.

Meanwhile, the present invention aims to confirm the effect of boar-specific pheromones on improving the reproductive performance of sows, mares, does, ewes nannies and cows, and to provide a composition for improving reproductive performance of livestock using the same.

### Disclosure of Invention

### Technical Problem

The present invention aims to provide a composition that can improve the reproductive performance of livestock in order to solve economic problems that may arise due to impaired reproductive performance in livestock, such as delayed return to estrus, increased frequency of weak estrus, and irregular estrus periods.

### Solution to Problem

The present invention provides a composition for improving reproductive performance of female livestock, including *para*-cresol (*p*-cresol) as an active ingredient, in which the improvement in reproductive performance shortens the number of days to return to estrus, induces estrus, or promotes estrus.

In the present invention, the composition may preferably comprise one or more types of waxes selected from the group consisting of paraffin wax, beeswax, wood wax, and carnauba wax, but is not limited thereto.

In the present invention, the composition may further include one or more selected from ethanol, purified water, and a surfactant, but is not limited thereto.

In the present invention, the *para*-cresol (*p*-cresol) may preferably be comprised in an amount of 0.01 wt% to 30 wt% based on the total composition, but is not limited thereto.

In the present invention, the female livestock may preferably be a sow, a mare, a doe, an ewe, a nanny, or a cow but is not limited thereto.

In the present invention, the composition may preferably be a solid or liquid, but is not limited thereto.

In addition, the present invention provides a method for improving reproductive performance of female livestock, which comprises mixing *para*-cresol (*p*-cresol) into one or more waxes selected from the group consisting of paraffin wax, beeswax, wood wax, and carnauba wax, in which the improvement in reproductive performance is shortening the number of days to return to estrus, or inducing or promoting estrus.

In the present invention, the composition is a mixture of 70 wt% to 99.99 wt% of wax and 0.01 wt% to 30 wt% of *para*-cresol (*p*-cresol), but is not limited thereto.

In the present invention, the composition for improving the reproductive performance of female livestock may be characterized by continuously releasing *para-*cresol (*p*-cresol), but is not limited thereto.

In addition, the present invention provides a method for improving reproductive performance of female livestock, which comprises applying the composition to a part of the body or a breeding place of the female livestock, or spraying it on the body, in which the improvement in the reproductive performance is shortening the number of days to return to estrus or inducing or promoting estrus.

### Advantageous Effects of Invention

The present invention provides a composition for improving reproductive performance of livestock that not only has the effect of improving productivity by improvement in the reproductive performance of livestock, but also prevents various problems that may occur due to the indiscriminate use of hormone preparations, and alleviates the economic burden of breeders, such as feed costs.

### Brief Description of Drawings

Fig. 1 is a diagram showing the results of SPME analysis of volatile organic compounds contained in the urine of a boar or sow, according to an embodiment of the present invention.
Fig. 2 is a diagram comparing the content of volatile organic compounds contained in the urine of a boar or sow, according to an embodiment of the present invention.
Fig. 3 is a diagram comparing the p-cresol content in the urine of a boar or sow, according to an embodiment of the present invention.
Fig. 4 is a diagram showing an example of a paraffin block comprising p-cresol, according to an embodiment of the present invention.
Fig. 5 is a diagram showing an example in which a paraffin block comprising p-cresol is placed in a weaning sow pen, according to an embodiment of the present invention.
Fig. 6 is a diagram showing an example in which a paraffin block comprising p-cresol is mounted on a halter of a mare, according to an embodiment of the present invention.
Fig. 7 is a diagram showing an example in which a paraffin block comprising p-cresol is mounted on a halter of a doe, according to an embodiment of the present invention.

### Best Mode for Carrying out the Invention

Hereinafter, the present invention will be described in detail through embodiments of the present invention with reference to the attached drawings. However, the following embodiments are provided as examples of the present invention, and detailed descriptions of techniques or configurations known to those skilled in the art may be omitted if it is deemed that such detailed descriptions would unnecessarily obscure the essence of the invention, the present invention is not limited thereby. The present invention is capable of various modifications and applications within the description of the patent claims described below and the scope of equivalents interpreted therefrom.

In addition, the terms used herein are those used to appropriately express preferred embodiments of the present invention, and may vary depending on the intention of the user or operator or the customary practices in the field to which the present invention belongs. Therefore, the definitions of these terms should be made based on the overall context of this specification. Throughout the specification, when a part is described as "comprising" a certain element, this means that it may further include other elements rather than excluding other elements, unless specifically stated to the contrary.

Throughout this specification, "%" used to indicate the concentration of a specific substance is (w/w)% for solid/solid, (w/v)% for solid/liquid, and (v/v)% for liquid/liquid.

Hereinafter, the present invention will be described in more detail.

In an aspect, the present invention provides a composition for improving reproductive performance of female livestock comprising para-cresol (p-cresol) as an active ingredient.

The *para*-cresol (*p*-cresol) is also known as 4-methylphenol and refers to a compound represented by the following Formula 1:

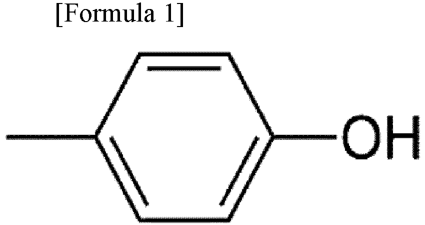

In the present invention, the improvement in reproductive performance of livestock may include shortening the number of days in which estrus recurs, or inducing or promoting estrus.

In the present invention, the composition for improving fertility comprising *para-*cresol (*p*-cresol) may be solid or liquid.

For example, in the case of a solid composition, the wax may be completely melted to prepare a liquid and then mixed with para-cresol (p-cresol) to solidify the same. In the case of a solid composition, it may comprise 0.01 wt% to 30 wt% of*p-*cresol, preferably 1 wt% to 20 wt%, and more preferably 1 wt% to 10 wt%, based on the total weight of the composition, but is not limited thereto. The content of*p*-cresol may be adjusted depending on the desired estrus-inducing effect.

For example, in the case of a liquid composition, it may be a spray formulation of a liquid composition in which ethanol, purified water, and *p*-cresol are mixed, or a fragrance (diffuser) formulation of the liquid composition in which ethanol, purified water, and *p*-cresol are mixed. In the case of the liquid composition, it may comprise 0.01 wt% to 30 wt% of *p*-cresol, preferably 1 wt% to 20 wt%, and more preferably 1 wt% to 10 wt%, based on the total weight of the composition, but is not limited thereto. The content of *p*-cresol may be adjusted depending on the desired estrus-inducing effect.

In an aspect, the present invention provides a method for preparing a composition for improving reproductive performance of livestock, which comprises mixing *para-*cresol *(p*-cresol*)* with one or more waxes selected from the group consisting of paraffin wax, beeswax, wood wax, and carnauba wax.

In an aspect, the present invention provides a method for improving reproductive performance of livestock, which comprises applying the composition to a part of the body or a breeding place of the female livestock or spraying the same on the body.

In the present invention, the method for improvement in reproductive performance of livestock is preferably to allow livestock to inhale *para*-cresol (*p-*cresol) diffused from the composition.

In the present invention, the wax is preferably in a liquid state. It is preferred that the wax is prepared from a solid state by boiling the wax to a completely liquid state and mixing it with *para*-cresol (*p*-cresol).

Meanwhile, research has reported that the GnRH hormone secretion wave generator located in the hypothalamus plays a crucial role in regulating reproductive function in female livestock, and that FSH and LH hormones produced by stimulation of GnRH promote follicular growth and cause ovulation of eggs (T Hamada et al., "Pheromone-induced stimulation of hypothalamic gonadotropin-releasing hormone pulse generator in ovariectomized, estrogen-primed goats", Neuroendocrinology. 1996 Oct;64(4):313-9.; Robert V Knox, "Recent advancements in the hormonal stimulation of ovulation in swine", Vet Med (Auckl). 2015; 6: 309-320.).

In one embodiment of the present invention, it was confirmed that *para*-cresol (*p-*cresol) of the present invention shortens the return to estrus after parturition in post-weaned sows, which is the result of stimulating the secretion of FSH and LH hormones by the sow's GnRH hormone, and it is expected that the inhalation of *para*-cresol (*p-*cresol) will have the effect of inducing follicle development in sows and stimulating the ovulation of multiple oocytes, thereby increasing the litter size.

### Exemplary Embodiments of the Invention

Hereinafter, the present invention will be described in more detail through examples. These examples are intended to explain the present invention more specifically, and the scope of the present invention is not limited to these examples.

### <Example 1> Analysis of volatile components comprised in pig urine

Urine was collected from two uncastrated adult boars and five sows, and the volatile components comprised in each urine were compared using the Solid Phase Microextraction (SPME) test.

For each subject, 2 mL of urine was extracted three times. After forming the septum of the sample container in a state where the fiber fully was inserted inside, the fiber was exposed to the outside and the analyte was extracted using the headspace method for about 1 hour. After the extraction, once the fiber retracted, it was exposed in the GC inlet and the adsorbed analytes were thermally desorbed at high temperature. Then, the analytes were automatically injected into the analysis column and numerical analysis was performed for each analyte.

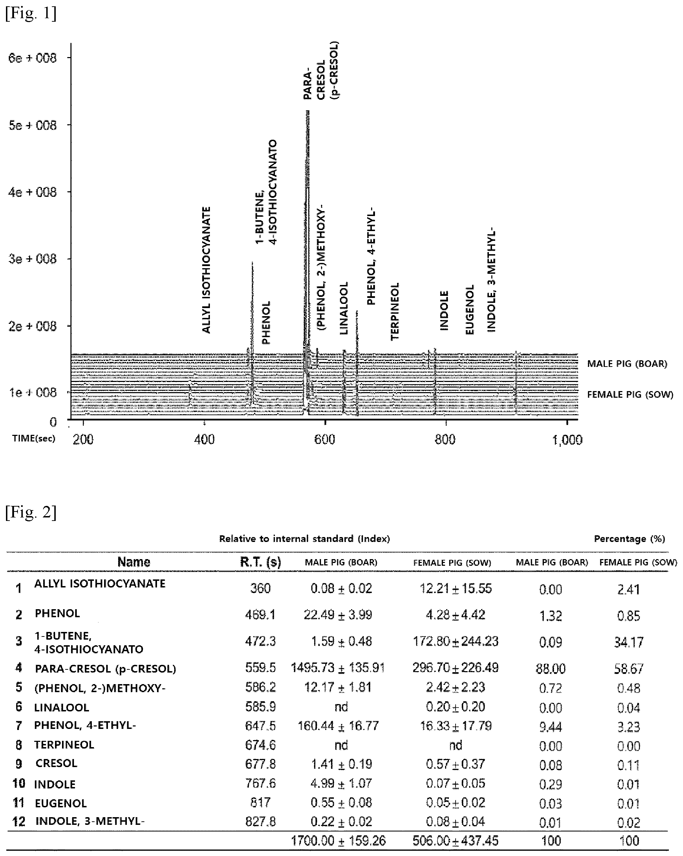

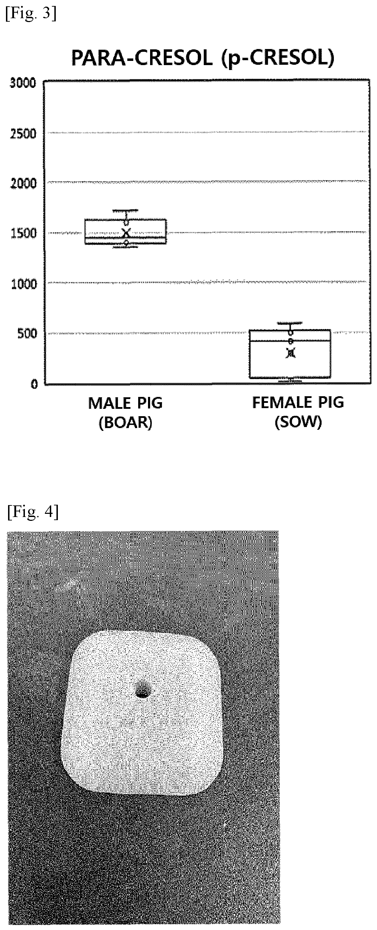

Fig. 1 is a diagram showing the results of SPME analysis, and Fig. 2 is a diagram comparing and analyzing the content of volatile organic compounds comprised in each urine. While a large amount of *p*-cresol was detected in boars, a relatively small amount of *p*-cresol was detected in sows. Fig. 3 is a diagram analyzing the content of *p*-cresol comprised in the urine of boars and sows.

### <Example 2> Preparation of paraffin blocks comprising p-cresol

95 g of completely melted paraffin wax and 5 g of *p*-cresol were mixed with stirring. The mixture was poured into a wax tray and completely hardened to prepare a paraffin block (X-factor pheromone block) that continuously emits *p*-cresol. Fig. 4 is a diagram showing the appearance of the paraffin block.

### <Example 3> Confirmation of effect of paraffin blocks comprising p-cresol on improving reproductive performance of sows

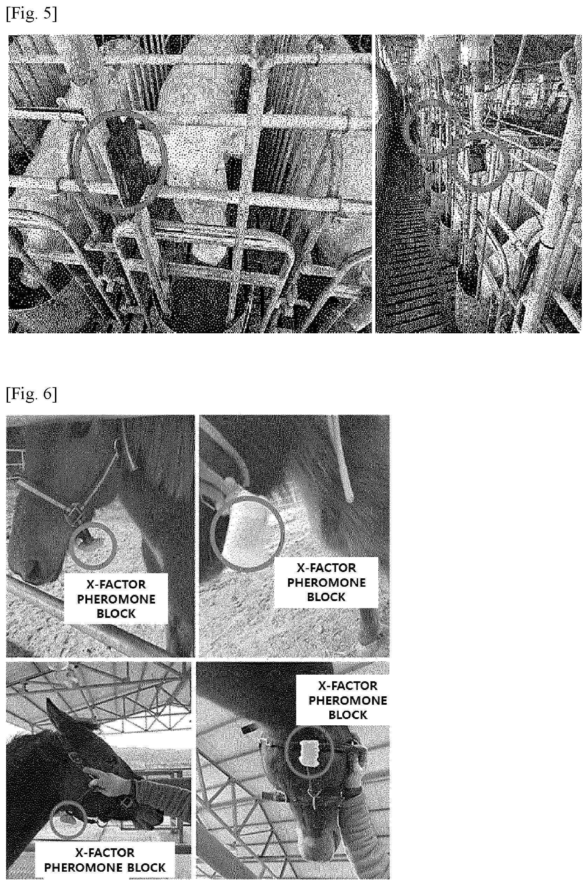

(Experiment 1) In order to confirm the effect of paraffin blocks comprising *p-*cresol on the litter size at the time of the next birth of sows, an experiment was performed for about 8 months from August 2022 to April 2023. As a treatment method, the paraffin blocks comprising 5% *p*-cresol prepared in Example 2 were placed in a weaning sow pen (Fig. 5). Among 270 farrowing sows, 143 were set as control groups and reared without paraffin blocks. The experimental results are shown in Table 1 below. The litter size refers to the number of piglets born from one sow.

**[Table 1]**

| | Time Required to Return to Estrus after Weaning (Days) | Time Required to Return to Estrus after Weaning (Hours) | Litter Size (Average) |
|---|---|---|---|
| Control group (n = 143) | 4.1 | 98.4 | 12.5 |
| 5% *p*-cresol (n = 127) | 3.7 | 88.8 | 13.5 |

As shown in Table 1 above, it was confirmed that the paraffin blocks comprising *p*-cresol of the present invention can improve the reproductive performance of sows by increasing the average litter size.

(Experiment 2) In order to confirm the effect of paraffin blocks comprising *p-*cresol on the return to estrus after weaning of sows, an experiment was performed for about 10 months from October 2022 to August 2023. As a treatment method, paraffin blocks comprising 5% *p*-cresol were placed in a weaning sow pen (Fig. 5). Among 926 farrowing sows, 468 were set as control groups and reared without paraffin blocks. The experimental results are shown in Table 2 below. The normal estrus rate refers to the proportion of all experimental animals that come into estrus within 5 days after weaning.

**[Table 2]**

| | Time Required to Return to Estrus after Weaning (Days) | Time Required to Return to Estrus after Weaning (Hours) | Normal Estrus Rate (%) |
|---|---|---|---|
| Control group (n = 468) | 4.4 | 105.6 | 84.5 |
| 5%*p*-cresol (n = 458) | 4.0 | 96 | 91.4 |

As shown in Table 2, it was confirmed that when the paraffin blocks comprising *p*-cresol of the present invention were placed in a sow pen, the time required to return to estrus was shortened and the normal estrus rate was increased. These results indicate that continuous exposure of *p*-cresol to sows has the effect of shortening the number of days to return to estrus, thereby improving the reproductive performance of sows.

(Experiment 3) In order to compare the effect of paraffin blocks comprising *p-*cresol and an estrus inducing injection (Corpulin) on the return to estrus of sows after weaning, an experiment was performed in June 2023. As a treatment method, paraffin blocks comprising 5% *p*-cresol were placed in a weaning sow pen (Fig. 5). Among the 464 farrowing sows, 224 were set as positive controls (sows with injections) and reared without paraffin blocks. The experimental results are shown in Table 3 below. The litter size refers to the total number of farrowings in a sow, and the normal estrus rate refers to the proportion of all experimental animals that come into estrus within 5 days after weaning.

**[Table 3]**

| | Litter Size (Average) | Time Required to Return to Estrus after Weaning (Days) | Normal Estrus Rate (%) |
|---|---|---|---|
| Control group (n = 224) | 4.7 | 3.9 | 86.6 |
| 5%*p*-cresol (n = 240) | 4.1 | 3.5 | 95.0 |

As shown in Table 3 above, it was found that when the paraffin blocks comprising p-cresol of the present invention were placed in a sow pen, the time required to return to estrus was shortened and the normal estrus rate was increased, compared to using the injection (Corpulin). These results indicate that continuous exposure of *p*-cresol to sows has the effect of shortening the number of days to return to estrus, thereby improving the reproductive performance of sows.

### <Example 4> Confirmation of effect of paraffin blocks comprising p-cresol on improving reproductive performance of mares

January and February are the non-breeding months for mares. In this experiment, an X-factor pheromone block corresponding to the paraffin block comprising 5% *p-*cresol prepared in Example 2 above was mounted on mares' halters over two months (January and February) to determine whether the mares' reproductive performance could be improved during the non-breeding season (Fig. 6). Among the six mares, three served as controls and were mounted with paraffin blocks without *p*-cresol. The experimental results are shown in Table 4 below.

**[Table 4]**

| | Time Required to Estrus after Mounting (Days) | Estrus Induction Rate (%) |
|---|---|---|
| Control group (n = 3) | none | none |
| 5%*p*-cresol (n = 3) | 4.7 | 100% |

As shown in Table 4 above, the control group did not exhibit estrous behavior during the non-breeding season, whereas mares with paraffin blocks comprising *p-*cresol in their halters, *i.e.,* those exposed to *p*-cresol continuously, began estrus within 3, 6, and 5 days, respectively, despite being in the non-breeding season, with an average of 4.7 days after mounting. These results indicate that continuous exposure of mares to *p*-cresol has the effects of inducing and promoting estrus, thereby improving the reproductive performance of mares.

### <Example 5> Confirmation of effect of paraffin blocks comprising p-cresol on improving reproductive performance of does

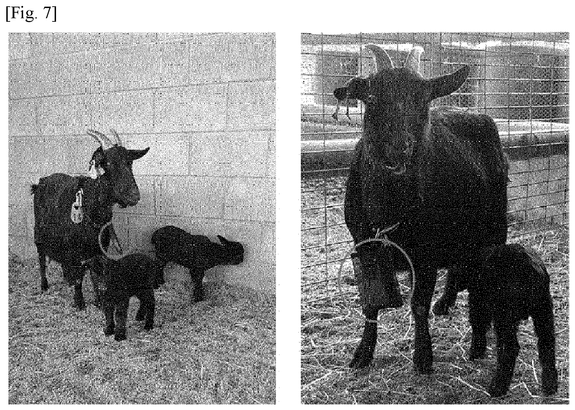

In order to determine the effect of paraffin blocks comprising *p*-cresol on the return to estrus after parturition of does, an experiment was performed for one month in January 2023. As a treatment method, the paraffin block comprising 5% *p*-cresol prepared in Example 2 was mounted on the halters of does immediately after parturition (Fig. 7). Among the six calving does, three were set as a control group and were mounted with paraffin blocks without *p*-cresol. The experimental results are shown in Table 5 below.

**[Table 5]**

| | Time Required to Estrus after Mounting (Days) |
|---|---|
| Control group (n = 3) | 29 |
| 5% Pheromone Block (n = 3) | 16.25 |

As shown in Table 5 above, in the control group, the first estrus was observed 29 days after mounting, but in the does on which a paraffin block comprising *p*-cresol was mounted on their halters, that is, those which were continuously exposed to *p*-cresol, the first estrus was observed 16.25 days on average after the mounting. These results indicate that continuous exposure of does to *p*-cresol has the effects of inducing and promoting estrus, thereby improving the reproductive performance of does.

## Claims

1. A composition for improving reproductive performance of female livestock comprising *para*-cresol (*p*-cresol) as an active ingredient, wherein the improvement in reproductive performance is shortening the number of days to return to estrus, or inducing or promoting estrus.

2. The composition of claim 1, wherein the composition comprises one or more waxes selected from the group consisting of paraffin wax, beeswax, wood wax, and carnauba wax.

3. The composition of claim 1, wherein the composition further comprises one or more selected from ethanol, purified water, and a surfactant.

4. The composition of claim 1, wherein the *para*-cresol (*p*-cresol) is comprised in an amount of 0.01 wt% to 30 wt% relative to the total composition.

5. The composition of claim 1, wherein the female livestock is a sow, a mare, a doe, an ewe, a nanny, or a cow.

6. The composition of claim 1, wherein the composition is a solid or liquid.

7. A method for preparing a composition for improving the reproductive performance of female livestock comprising mixing *para*-cresol (*p*-cresol) into one or more waxes selected from the group consisting of paraffin wax, beeswax, wood wax, and carnauba wax, wherein the improvement in reproductive performance is shortening the number of days to return to estrus, or inducing or promoting estrus.

8. The method of claim 7, wherein the composition is a mixture of 70 wt% to 99.99 wt% of wax and 0.01 wt% to 30 wt% of *para*-cresol (*p*-cresol).

9. The method of claim 7, wherein the composition for improving the reproductive performance of female livestock is **characterized by** continuously releasing *para*-cresol (*p*-cresol).

10. A method for improving the reproductive performance of female livestock, comprising applying the composition of any one of claims 1 to 6 to a part of the body of the female livestock or a breeding place or spraying the same on the body, wherein the improvement in reproductive performance is shortening the number of days to return to estrus, or inducing or promoting estrus.
